# FASCICULE DE BREVET EUROPEEN

(11) **EP 1 108 061 B1**
(45) Date de publication et mention de la délivrance du brevet: **14.05.2003**
(21) Numéro de dépôt: 99941696.9
(22) Date de dépôt: 06.09.1999
(51) Int. Cl.: C12Q 1/68

(54) **PROCEDE D'AMPLIFICATION DE LA TOTALITE DES FRAGMENTS D'ADN D'UN ECHANTILLON**
VERFAHREN ZUR VERVIELFÄLTIGUNG DER KOMPLETTEN DNA IN EINER PROBE
METHOD FOR AMPLIFYING ALL DNA FRAGMENTS IN A SAMPLE

(30) Priorité: 04.09.1998 FR 9811081
(43) Date de publication de la demande: 20.06.2001
(73) Titulaire: Genolife, Saint-Beauzire 63360 Gerzat (FR)
(72) Inventeur: PROVOT, Christian, F-63670 Le Cendre (FR); CHAUBRON, Franck, F-63400 Chamalières (FR); MARTIN, Anne-Céline, F-63400 Chamalières (FR)
(74) Mandataire: Warcoin, Jacques
(86) Numéro de dépôt international: FR9902113
(87) Numéro de publication internationale: WO00014277

(56) Documents cités:
- EP-A- 0 778 351
- WO-A-95/11995
- WO-A-97/08185
- WO-A-97/27317
- US-A- 5 162 209
- TOELLNER K -M ET AL: "The use of reverse transcription polymerase chain reaction to analyse large numbers of mRNA species from a single cell" JOURNAL OF IMMUNOLOGICAL METHODS, vol. 191, no. 1, 10 mai 1996 (1996-05-10), page 71-75 XP004020856
- WEXLER A ET AL: "A PROCEDURE TO AMPLIFY CDNA FROM DSRNA TEMPLATES USING THE POLYMERASE CHAIN REACTION" METHODS IN MOLECULAR AND CELLULAR BIOLOGY,US,NEW YORK, NY, vol. 2, 1991, page 273-279 XP000749613 ISSN: 0898-7750
- CORMACK R S ET AL: "Rapid amplification of genomic ends (RAGE) as a simple method to clone flanking genomic DNA" GENE: AN INTERNATIONAL JOURNAL ON GENES AND GENOMES,GB,ELSEVIER SCIENCE PUBLISHERS, BARKING, vol. 194, no. 2, page 273-276 XP004093354 ISSN: 0378-1119
- JANSSEN P ET AL: "Evaluation of the DNA fingerprinting method AFLP as a new tool in bacterial taxonomy" MICROBIOLOGY, vol. 142, no. 142, juillet 1996 (1996-07), pages 1881-1893, XP002097675
- SEDLAK B J: "GENE CHIP TECHNOLOGY READY TO IMPACT DIAGNOSTIC MARKETS" GENETIC ENGINEERING NEWS, 1 décembre 1997 (1997-12-01), page 1, 14, 34 XP000749581

## Description

La présente invention concerne un procédé de caractérisation de la totalité des fragments d'ADN d'un échantillon, lesdits fragments étant de faibles tailles et se trouvant souvent abîmés et/ou à l'état de traces notamment dans les produits finis ou transformés. L'invention réside dans la pré-amplification de la totalité des fragments d'ADN avant une éventuelle amplification spécifique, et donc le procédé est particulièrement adapté à l'analyse, par hybridation moléculaire, en particulier sur puces à ADN, de la totalité de l'ADN provenant de tout type d'échantillon éventuellement d'une importante complexité et/ou ayant subi des traitements dénaturants.

Diverses méthodes de caractérisation d'un échantillon complexe d'un point de vue biochimique, basées sur l'identification de séquences nucléotidiques, sont utilisées notamment dans le domaine agro-alimentaire. Ces méthodes peuvent s'appliquer aux matières premières telle qu'une simple graine, cependant elles montrent certaines limitations quant à l'analyse d'un produit contenant de nombreux éléments entrant dans sa composition et/ou ayant subi des traitements qui dénaturent les molécules à caractériser. Il est donc nécessaire d'enrichir et/ou de purifier le matériel contenu dans un échantillon d'intérêt si celui-ci possède des constituants traités ou conditionnés provenant d'origine diverses, comme c'est le cas pour les produits finis.

Une fois le matériel nucléique obtenu, à un degré de pureté suffisant pour permettre son analyse, il doit être soumis à un ou plusieurs tests destinés à le caractériser par hybridation moléculaire. Toutefois, ces méthodes sont généralement restreintes aux laboratoires d'analyse du fait, soit de l'utilisation de radioactivité (bien que des méthodes comme la luminescence tendent à remplacer la radioactivité), soit du trop faible nombre d'échantillons analysables simultanément. Dans certains cas, ces techniques ne peuvent pas être utilisées du fait d'un manque de sensibilité, ce manque étant essentiellement dû à la faible quantité, et/ou à la mauvaise qualité, du matériel nucléique récupéré dans l'échantillon

La technique la plus couramment utilisée est la PCR (Polymerase Chain Reaction) Cette méthode permet d'amplifier spécifiquement, au cours de nombreux cycles réactionnels (de l'ordre de 25 à 45) un acide nucléique compris entre 2 amorces spécifiques de séquences nucléotidiques connues Ces amorces sont des oligonucléotides de l'ordre de 15 à 40 bases, dont la séquence s'apparie parfaitement avec les séquences flanquantes de la séquence à amplifier Il est classique, à partir d'un échantillon nucléique, de n'amplifier qu'une seule séquence.

Des PCR en "multiplex" ont été décrites dans [Apostolakos (1993) Anal Biochem, 213, 277-284] Dans des conditions particulières, il est possible dans un même tube réactionnel d'amplifier plusieurs séquences simultanément en utilisant plusieurs couples d'amorces Le nombre de couples d'amorces dépasse rarement 3. En effet, au-delà, les amplifications perdent leur spécificité (apparition de produits d'amplification non attendus) ou une ou plusieurs amplifications ne fonctionnent pas ou peu, bien qu'un exemple de l'amplification en multiplex de 9 séquences ait été décrit [Edwards (1994) PCR Methods Applic., 3, S65-S75]

D'autres techniques, dérivées plus ou moins de la PCR, ont été développées
- La LCR (Ligase Chain Reaction), basée sur l'utilisation d'une ADN ligase thermostable [Barany (1991) Proc. Natl Acad Sci USA, 88, 189-193]
- La Gap-LCR est dérivée de la LCR
- L'ERA (End Run Amplification) est développée par Beckman Instruments, son dérivé étant la GERA (Gap-ERA) [Adams (1994) Novel amplification technologies for DNA/RNA-based diagnostics meeting, San-Francisco, CA, USA]
- La CPR (Cycling Probe Reaction), qui met en oeuvre un chimère ADN-ARN-ADN et la ribonucléase H [Duck (1990) BioTechniques, 9, 142-147] et est développée par la société ID Biochemical Corporation
- La SDA (Strand Displacement Amplification) [Walker (1992) Nucleic Acids Res., 20, 1691-1696], brevetée par la société Becton Dickinson, qui autorise une analyse en multiplex [Walker (1994) Nucleic Acids Res., 22, 2670-2677] Cependant il est difficile d'analyser plus de 3 séquences simultanément par cette méthode.
- La TAS (Transcription-based Amplification), [Kwoh (1989) Proc. Natl Acad Sci. USA, 86, 1173-1177], utilise la Reverse Transcriptase et la T7 polymerase. La Self Sustained Sequence Replication s'apparente à la TAS [Gingeras (1990) Ann. Biol Clin.,48, 498-501].
- La NASBA (Nucleic Acid Sequence-Based Amplification) est assez similaire à la 3SR [Kievits (1991) J Virol Methods, 35, 273-286].
- Enfin, les propriétés de la Qβ replicase (ARN polymérase-ARN dépendante isolée du bactériophage Qβ) ont été évoquées avant la PCR [Haruna (1965) Proc Natl. Acad. Sci USA, 54, 579-587] et cette enzyme a été utilisée en technique d'amplification à partir de 1983 [Miele (1983)J Mol. Biol, 171,281 295].

Il apparaît au vu des documents cités supra, qu'il n'est pas possible de caractériser des centaines, et à plus forte raison des milliers de séquences nucléotidiques contenues dans une solution d'ADN, en un nombre d'étapes restreint.

Il est néanmoins possible d'amplifier, dans un nombre d'étapes limité, et en utilisant un nombre d'amorces important (supérieur au nombre utilisé en multiplex), la quasi totalité de l'ADN contenu dans un extrait

Une des approches pourraient être la technique AFLP (Amplified Restriction Flagment Polymorphism ou Amplified Fragment Lenght Polymorphismn) qui consiste en l'utilisation d'enzymes de restriction pour digérer l'ADN en des sites précis, puis d'adapteurs se fixant précisément à ces sites de coupures, apportant en plus une séquence d'ADN suffisante pour permettre ensuite l'hybridation d'amorces. Dans un procédé commercialisé par exemple par la société Gibco-BRL, les enzymes EcoRI et MseI sont utilisés, puis 8 adapteurs/amorces de chaque site de coupure sont utilisés pour l'étape d'amplification.

Cette méthode est toutefois restreinte à l'analyse d'ADN d'assez bonne qualité (généralement directement extrait d'un tissu ou d'un organisme). En effet, il est nécessaire pour que les amplifications aient lieu que les 2 adapteurs soient présents aux extrémités de l'ADN digéré, donc que l'ADN ait été digéré à ces sites. Dans le cas d'ADN issu d'un produit transformé, la taille de cet ADN est de l'ordre de quelques centaines de paires de bases (200 à 400). La probabilité de présence d'un site EcoRI (reconnaissant un site composé du palindrome de 6 paires de bases; GAATTC) est de 1/4⁶, soit 1 site potentiel pour 4096 paires de bases

Les enzymes de restriction reconnaissant les sites les plus fréquents (4 paires de bases), comme MseI, vont par contre statistiquement couper l'ADN toutes les 256 paires de bases Comme il est nécessaire de couper l'ADN 2 fois pour générer les 2 sites d'amorçage de la PCR, la probabilité de générer ces 2 sites, sur un fragment de quelques centaines de paires de bases est faible et les produits d'amplification ne reflètent pas l'ensemble de l'ADN de départ

De courtes séquences répétées, dispersées tout le long du génome, appelées "microsatellites", ont été utilisées pour amplifier, à l'aide d'amorces complémentaires à ces séquences microsatellites, les séquences comprises entre celles-ci [Zietkeiwicz (1994) Genomics, 20, 176 183; Weising (1995) PCR Methods Applic., 4, 249-255] Ce type d'amplification permet surtout de réaliser du typage génétique "fingerprinting" basé sur une analyse qualitative des produits d'amplification [Thomas (1993) Theor Appl Genet 86, 985-990] Une forte proportion du génome peut ainsi être amplifiée, mais pas sa totalité, en particulier du fait que certaines séquences microsatellites sont trop éloignées pour permettre une amplification PCR

Trois méthodes ont été décrites, revendiquant l'amplification non spécifique de toutes les séquences nucléotidiques d'un échantillon [Lüdecke (1989) Nature, 338, 348-350 ou Kinzler (1989) Nucleic Acids Res, 17, 3645-3653, Zhang (1992) Proc Natl Acad; USA, 89, 5847-5851; et Grothues (1993) Nucleic Acids Res, 21, 1321-1322, et US 5,731,171].

Le principe de cette dernière méthode, s'inspirant des 2 autres et revendiquant une amélioration technique, repose sur l'utilisation d'un très grand nombre d'oligonucléotides de 10 à 20 bases, représentant toutes les séquences possibles, auxquels une séquence spécifique est associée en 3' Au cours d'une première PCR, avec un petit nombre de cycles, ces oligonucléotides vont théoriquement s'apparier sur toutes les séquences et les cycles d'amplification vont incorporer la séquence spécifique à tous les fragments amplifiés Après une filtration sur gel, ayant pour but de séparer les oligonucléotides libres de l'ADN, une seconde PCR utilisant un oligonucléotide complémentaire de la séquence spécifique est réalisée sur l'ADN. Selon les auteurs, ce procédé permet l'amplification de la totalité de l'ADN sur des échantillons de tailles variant de 400 paires de bases à 40 mégabases Cependant, les oligonucléotides pouvant s'hybrider à toutes les séquences possibles, sur un même brin d'ADN, ne s'hybrident pas forcément aux extrémités et donc le fragment entier n'est pas amplifié De surcroît, la température d'hybridation est de 30°C en ce qui concerne la première PCR avec ces oligonucléotides aléatoires (température en dessous de laquelle il est difficile de descendre avec les appareils à PCR). La température d'hybridation d'un oligonucléotide étant calculée sur la base de 2°C par A ou T et de 4°C pour G ou C, un oligonucléotide constitué d'une combinaison de 10 A ou T s'hybride à 20°C et donc ne s'hybridera pas, ou très mal à 30°C. En utilisant une telle température, la richesse en A/T de l'oligonucléotide ne devrait pas excéder 50% de la séquence. Ce problème est d'ailleurs soulevé dans cette publication, qui précise en outre qu'un nombre de séquences est au minimum sous représentées et donc implicitement que certaines séquences ne sont pas amplifiés Le risque de n'amplifier que partiellement une séquence particulière augmente de façon inversement proportionnelle à la quantité de cette séquence dans le milieu de départ et l'apparition de faux négatifs est un problème pour l'analyse fiable d'un échantillon

L'amplification des ARNm totaux de cellules a été décrite notamment de la page 120 à 121 de "La PCR, un procédé de réplication in vitro", Daniel Larzul, Collection Génie Génétique, Ed Lavoisier Cette méthode bénéficie du fait que la majorité des ARNm possède une queue poly(A) à l'extrémité 3' Un oligonucléotide poly(dT) sert d'amorce lors de la première étape d'amplification, puis un poly(dG) est ajouté à l'extrémité 3' du brin nouvellement synthétisé par la terminale transférase, et sert de site d'ancrage pour une amorce poly(dC)

Le document US 5,162,209 décrit un procédé qui permet la génération d'ADN double brin à partir d'ARNm. Bien que l'amplification des ARNm totaux possède certaines caractéristiques assez similaires à la présente invention, cette technique n'est pas a priori directement applicable pour accomplir l'objectif de la présente invention En effet, après dénaturation des brins obtenus par élongation à partir de poly(dC), on fait agir la taq DNA polymérase avec une amorce poly(dG). On obtient ainsi deux produits qui ne sont pas amplifiables puisque deux nouvelles amorces poly(dC) aurait une polarité 3' - 5', ce qui ne peut pas servir de susbtrat aux polymérases La méthode selon l'invention permet de surmonter cette difficulté notamment grâce à la réalisation d'une dénaturation/renaturation lente qui autorise l'appariement des deux brins 5'-poly(dG)-séquence à amplifier -3' ou grâce à une seconde étape mettant en oeuvre une terminale transférase. La polymérase synthétise des extrémités poly(dC) qui servent ensuite d'ancrage aux amorces poly(dG) qui possèdent cette fois la bonne polarité 5' - 3' Toutefois, la technique de l'invention n'est valable que pour l'amplification de brins relativement petits On peut d'ailleurs signaler que les techniques décrites dans US 5,162,209 et WO 97/08185, basées sur le fait que l'ARNm possède naturellement une queue poly(A), ne peuvent servir de point de départ pour une amplification PCR, car lesdites techniques, davantage destinée au clonage, ne produisent pas de produits amplifiables

La détection de produit d'ADN amplifié, par PCR ou d'autres méthodes, fait généralement appel à une analyse électrophorétique Des méthodes de détection en microplaques 96 puits ont également été décrites. Le produit d'amplification PCR est dénaturé et hybridé dans un puits de microplaque sur lequel est fixé un oligonucléotide de capture [Running (1990) BioTechniques, 8, 276-277] ou un ADN simple-brin contenant une séquence de capture [Kawai (1993) Anal Biochem, 209, 63-69]. Une au moins des amorces utilisées dans la PCR est par exemple biotinylée et la détection de l'hybridation est réalisée par addition de streptavidine couplée à une enzyme telle que la peroxydase, puis d'un substrat chromogène de l'enzyme

Des variantes commerciales de ce test utilisent des détections autres telles que la fluorescence. En utilisant un oligonucléotide de capture fixé sur les puits, une amorce de PCR biotinylée et un standard interne d'amplification, il a même été possible de réaliser de la PCR quantitative [Berndt (1995) Anal. Biochem., 225, 252-257]. La technique de capture de produits d'ADN amplifiés n'est pas restreinte à la PCR puisqu'elle a par exemple été adaptée, par la société Applied Biosystems, à la détection de Micobacterium tuberculosis par LCR [Winn-Deen (1993) Mol. Cell. Probes, 7, 179 186].

Ces méthodes, quantitatives ou non, ne renseignent, à partir d'une PCR, que sur la présence ou l'absence d'une séquence ADN cible au départ Une variante permet à partir d'une seule PCR sur le locus HLA-DR d'identifier, d'une façon semi-automatique, 30 typages différents grâce à l'utilisation de 20 sondes oligonucléotidiques de capture et 2 sondes de détection couplées à la péroxydase [Cros (1992) Lancet, 340, 870-873] Un test similaire, également appliqué au typage HLA, est commercialisé par la société Perkin Elmer

Parallèlement à l'utilisation de microplaques, des puces à ADN (ou "DNA chips"), destinées à identifier des séquences d'ADN, ont été décrites et commercialisées Le principe de cette technique consiste à identifier des séquences d'acide nucléique (ADN ou ARN) dans un échantillon sur la base d'une hybridation moléculaire La puce porte, greffée sur une surface adéquate, des centaines ou milliers d'oligonucléotides d'intérêt L'ADN de l'échantillon est dénaturé et mis dans des conditions d'hybridation avec la puce Toutefois, deux contraintes principales apparaissent dans ce procédé
(1) le phénomène d'hybridation doit pouvoir être détecté,
(2) la quantité d'ADN hybridée doit répondre aux contraintes de sensibilité du système de détection

Pour répondre à la première contrainte, l'ADN est généralement marqué à l'aide d'un marqueur fluorescent. Il faut néanmoins citer, comme alternative, le système de détection GENFET. il s'agit d'un dispositif d'une constitution proche de celle d'un transistor à effet de champ (FET) développé au laboratoire de physico-chimie des interfaces (Ecole Centrale, Lyon, France) L'hybridation dans ce cas conduit à une modification de la densité de charge du semi-conducteur à l'interface du semi-conducteur à l'interface Si et SiO₂, cette modification étant mesurée

Pour répondre à la seconde contrainte, l'ADN de l'échantillon est généralement amplifié par PCR Dans ce cas, l'utilisation d'amorces d'amplification couplées, par exemple à un marqueur fluorescent, ou l'incorporation de nucléotide(s) fluorescent(s) répond aux 2 contraintes simultanément. Néanmoins, l'intérêt de la puce réside dans sa capacité à fournir à partir d'un échantillon d'ADN des centaines ou milliers d'informations. La recherche de mutations ponctuelles en est un bon exemple. On hybride un produit d'amplification sur une puce comprenant de nombreuses séquences de capture nucléotidiques contenues dans le fragment amplifié, chaque séquence de capture différant par une base On peut ainsi, dans les conditions d'hybridation pour lesquelles seuls les produits d'amplification de séquence parfaitement complémentaire s'hybrident, déterminer, selon les séquences de capture hybridées quelle est la séquence du produit d'amplification et en déduire la présence d'une éventuelle mutation par rapport à un allèle de référence.

La puce présente alors un grand intérêt car le schéma général de l'expérimentation est: une préparation d'ADN - une amplification - une hybridation sur la puce. Dans ce schéma, l'analyse de l'étape "puce à ADN" porte sur une seule amplification de l'ADN (avec un seul couple d'amorces) car toutes les sondes de capture portées par la puce ont pour cibles un seul produit d'amplification.

Si on veut concevoir une puce portant des sondes de capture ayant pour cible des multiples produits d'amplification, il sera nécessaire, soit d'utiliser les techniques AFLP, microsatellites (ou une technique dérivée) pour amplifier la totalité de l'ADN génomique, soit de procéder à de multiples amplifications, ce qui, du fait du grand nombre de manipulations en amont de l'étape "puce", rend son intérêt en tant qu'outil de criblage ou caractérisation à haut débit peu intéressant Une amplification globale de l'ADN d'un échantillon, de type AFLP, serait a priori compatible avec les possibilités de criblage de la puce Or, l'AFLP n'est pas adaptée à l'amplification de petits fragments d'ADN contenus dans un échantillon complexe, tel que les produits finis agro-alimentaire

En conséquence, le procédé selon l'invention permet la caractérisation de l'ensemble des fragments d'ADN d'un échantillon, lesdits fragments étant de faibles tailles et se trouvant souvent abîmés et/ou à l'état de traces notamment dans les produits finis, ou transformés ou encore lors de prises d'échantillons en divers endroits De plus, le procédé de la présente invention, valable pour de l'ADN de petite taille, est également applicable à de l'ADN de grande taille dont on peut réduire la taille. L'avantage que procure l'invention réside dans la pré-amplification de la totalité des fragments d'ADN avant une éventuelle amplification spécifique, et donc le procédé est particulièrement adapté à l'analyse, par hybridation moléculaire, en particulier sur puces à ADN, de la totalité de l'ADN provenant de tout type d'échantillon éventuellement d'une importante complexité et/ou ayant subi des traitements dénaturants.

Ainsi, aucun document de l'art antérieur ne décrit, ni ne suggère la présente invention telle que définie ci-après

### Description de l'invention

La présente invention concerne un procédé d'amplification de la totalité des fragments d'ADN d'un échantillon, comprenant les étapes suivantes
a) Extraction de l'ADN et réduction le cas échéant de la taille des fragments d'ADN extraits, par cassure physique ou enzymatique de manière à obtenir une longueur moyenne comprise entre environ 25 et 500 pb
b) Addition d'un oligonucléotide poly(dX) aux extrémités 3' des fragments d'ADN par une terminale transférase.
c) Dénaturation et appariement avec une amorce poly(dY) complémentaire du poly(dX) de l'étape b)
d) Synthèse par une ADN polymérase en présence des quatre dNTP.

Les échantillons selon l'invention peuvent provenir de n'importe quelle source, de tout produit, matière ou substance, à l'état naturel ou qui ont subi des traitements, des transformations, et/ou des conditionnements. En ce qui concerne un nouvel échantillon dont l'état n'est pas connu à l'avance, l'expérimentateur peut, selon sa préférence, choisir une technique d'analyse de la longueur de fragments d'ADN parmi toutes les méthodes couramment utilisées dans le domaine technique

On entend par "le cas échéant", une situation dans laquelle les fragments extraits d'un échantillon ont conservés une taille assez importante (environ supérieure à 500 pb en moyenne). Il est alors nécessaire de procéder à une réduction de la taille des fragments Ce cas de figure peut survenir lors de l'analyse de matières premières non transformées.

On entend par « poly(dX) », « homopolymère poly(dX)» ou « oligonucléotide poly(dX) » dans le cadre de l'invention, un oligonucléotide de 11 à à 15 nucléotides de long, X désignant l'un des nucléotides dG, dC, dA, ou dT, ledit nucléotide pouvant éventuellement être modifié chimiquement. Cette séquence peut comporter en option un ou deux nucléotides aléatoires en son extrémité 3'. On entend par poly(dY) une séquence comprenant au moins une répétition de n'importe quelle base, ladite séquence étant complémentaire à poly(dX).

Avantageusement, le procédé décrit supra bénéficie d'une étape e) permettant l'obtention d'un ADN amplifiable par PCR. L'étape e) consiste en une dénaturation, puis en une lente renaturation pour apparier les brins complémentaires synthétisés à l'étape d) portant des extrémités protubérantes poly(dY) La renaturation lente est effectuée par un passage d'une température comprise entre 85°C et 105°C à une température comprise entre 45° et 25°C, de préférence de 95°C à 35°C avec une rampe de température allant environ de 0,5°C à 0,05°C par seconde, de préférence de 0,2°C par seconde Cette lente renaturation permet un réappariement des brins d'ADN et la formation de molécules d'ADN amplifiables par PCR
Une alternative équivalente à la renaturation lente consiste en une seconde étape de terminale transférase effectuée dans les mêmes conditions que l'étape b). Ceci permet de disposer d'un ADN avec une séquence homopolymérique poly(dX) aux 2 extrémités de l'ADN Cet ADN est soumis à une dénaturation puis une hybridation avec un poly(dY) et une polymérisation du brin complémentaire en présence d'une ADN polymérase.
L'ADN obtenu à l'étape e) peut dès lors être amplifié avec les étapes suivantes :
f) Synthèse par une ADN polymérase des poly(dX) complémentaires desdites protubérances , éventuellement en présence uniquement de dXTP
g) Succession de cycles de type PCR consistant en la dénaturation, l'appariement de l'amorce poly(dY), et la synthèse par une ADN polymérase

Il est avantageux dans le procédé de l'invention d'amplifier des fragments d'ADN qui possèdent une longueur moyenne comprise entre 100 et 300 pb, de préférence égale à 200 pb

3 L'addition d'un oligonucléotide poly(dX) aux extrémités 3' desdits fragments d'ADN telle que mentionnée à l'étape c) est effectuée de préférence par une terminale transférase Avant cette addition d'un oligonucléotide poly(dX) aux extrémités 3', des extrémités 3'-OH libres peuvent être générées par la nucléase P1 De même, il est possible de marquer l'oligonucléotide poly(dX) ou poly(dY) radioactivement, par un groupe fluorescent ou luminescent ou en utilisant un système permettant une révélation par colorimétrie, notamment le système biotine-streptavidine-couplée à une enzyme réagissant avec un substrat chromogène, fluorigène ou luminescent (par exemple la peroxidase). Une autre solution peut consister à incorporer au cours d'une des étapes de synthèse au moins un nucléotide marqué

Un aspect complémentaire de la présente invention concerne un procédé de caractérisation d'un échantillon consistant en l'hybridation des fragments d'ADN obtenus à partir d'un procédé tel que décrit supra sur une ou plusieurs séquences nucléiques de type ADN, ARN, ou PNA portée(s) sur un support solide, et en la visualisation du signal émis par le ou les fragment(s) hybridé(s). De préférence, le support solide peut être une puce à ADN, ARN ou PNA, une microplaque, ou un film, par exemple un film de nitrocellulose

Certaines de ces techniques de détection sont décrites en détail notamment dans [Running (1990) BioTechniques, 8, 276-277], [Kawai (1993) Anal Biochem , 209, 63-69], quantitative [Berndt (1995) Anal Biochem, 225, 252-257], [Winn-Deen (1993) Mol. Cell Probes, 7, 179 186], [Cros (1992) Lancet, 340, 870-873], incorporées dans la description par référence

Un autre aspect de l'invention a trait à un kit détection permettant de mettre en oeuvre le procédé décrit précédemment Ce kit peut comprendre notamment des oligonucléotides homopolymériques, une terminale transférase, la nucléase P1, et des sondes spécifiques permettant la détection des molécules recherchées A cet effet, le kit peut également comprendre des puces à ADN, ARN ou PNA permettant ladite détection Les agents pouvant entrer dans la composition du kit selon l'invention sont explicités plus en détail dans les exemples présentés ci-après

La présente invention vise également l'utilisation du procédé tel que mentionné supra pour l'identification d'un produit, d'une substance, et/ou d'une matière, à l'état naturel ou qui a subi des traitements, des transformations, et/ou des conditionnements, pour l'identification de son origine et/ou de son appartenance Par exemple, ce procédé ou le kit permet la détection de la présence d'organismes génétiquement modifiés (OGM) ou de traces d'OGM dans un échantillon.

Le procédé selon l'invention permet aussi l'identification et/ou la quantification de contaminants dans un produit, une substance, et/ou une matière, à l'état naturel ou qui a subi des traitements, des transformations, et/ou des conditionnements

Par exemple, le produit peut provenir du corps humain ou animal, il peut être une sécrétion humaine, éventuellement à l'état de trace Le produit peut-être un extrait végétal, ou animal, éventuellement transgénique Dans ce cas de figure le procédé de l'invention permet de détecter rapidement la présence de transgènes

Avantageusement, le produit peut-être un produit agro-alimentaire ou pharmaceutique et le contaminant un micro-organisme telle qu'une bactérie, un virus, ou un champignon.

Pour la suite de la description, on se référera aux légendes des figures présentées ci-après.

### Légendes

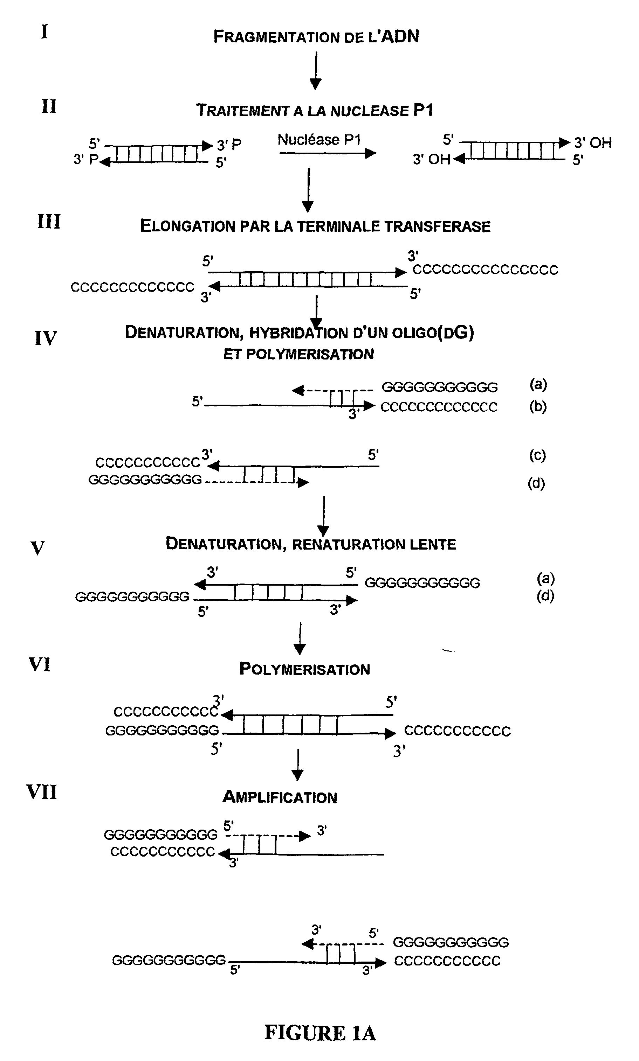

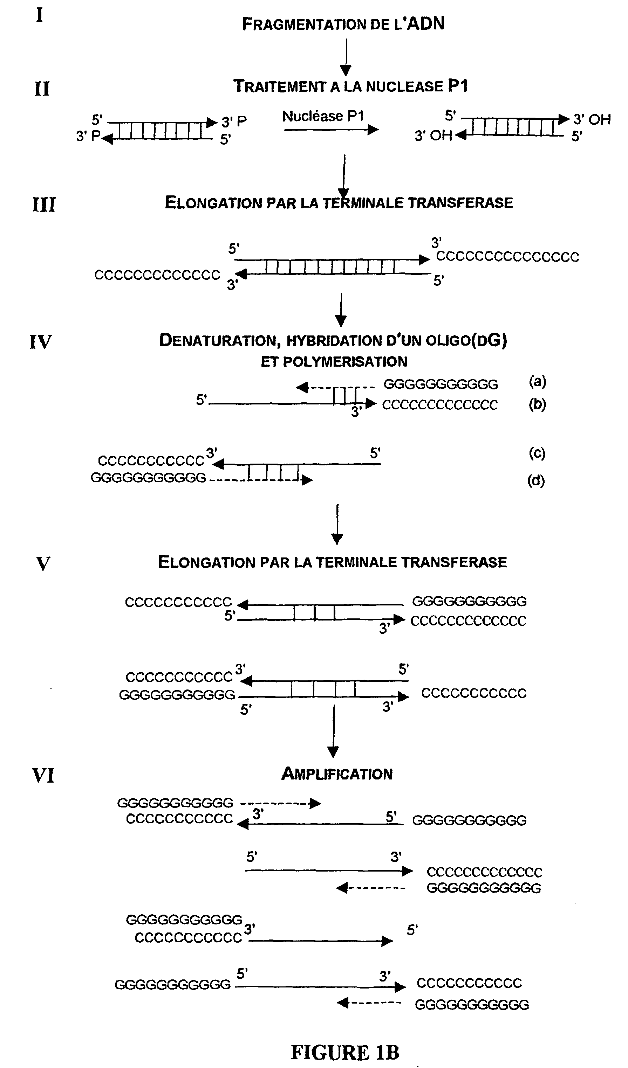

### Figures 1A et 1B : Représentations schématiques du procédé selon l'invention

Les flèches en trait épais indiquent les deux brins, en sens opposés, de l'ADN.
Les petits traits verticaux indiquent que les deux brins sont appariés.
5' et 3' définissent les extrémités de l'ADN selon la nomenclature.
Les C représentent l'addition de désoxycytidine-5'-monophosphate (le nombre de C ajouté n'est pas un paramètre dont dépend étroitement le succès de l'amplification L'appariement C:G n'est pas associé à des petits traits verticaux, cet appariement étant connu de l'homme du métier.
Les étapes V dans les figures 1A et 1B aboutissent au même résultat, à savoir permettre l'amplification aux étapes suivantes Cette étape consiste donc soit en une dénaturation-renaturation lente, soit en l'addition d'un oligonucléotide poly(dC) aux extrémités 3' des fragments d'ADN par une activité terminale transférase.

### Figure 2 : Mise en évidence de l'activité de la terminale transférase.

A. ADN génomique de soja fragmenté par soniquation et élongué par la terminale transférase.
B. ADN génomique de soja fragmenté par digestion enzymatique et élongué par la terminale transférase.

### Figure 3 : Révélation par électrophorèse sur gel d'agarose 2% des produits d'amplification obtenus après dénaturation et renaturation des ADN élongués.

A. ADN génomique de soja fragmenté par soniquation.
B. ADN génomique de soja fragmenté par digestion enzymatique.

### Figure 4 : Révélation par électrophorèse sur gel d'agarose 2% des produits d'amplification obtenus après 2 étapes de terminale transférase.

### Figure 5 : Amplification universelle d'ADN génomique. Révélation par hybridation des produits PCR obtenus après dénaturation/renaturation (1) ou 2 étapes de terminale transférase (2 et 3)

1. sonde spécifique d'un gène constitutif du soja
2 et 3. Deux sondes spécifiques de 2 gènes constitutifs du soja

### Description détaillée

Le procédé selon l'invention consiste à ajouter à tous les fragments d'ADN contenus dans un échantillon une chaîne constituée d'un seul type de nucléotide à l'extrémité 3' de cet ADN. Cette succession de nucléotides peut servir directement à la détection, après hybridation sur les sondes de capture portées par la puce, ou servir dans une étape suivante de base commune à tous les fragments pour un marquage ou une amplification.

L'enzyme préférentiellement utilisée est la terminale transférase (EC 27731; enzyme extraite en particulier du thymus de veau, également appelée terminale désoxynucléotidyl transférase). Cet enzyme a la propriété de catalyser, indépendamment de la séquence d'ADN, l'addition de désoxyribonucléosides triphosphates ("dNTP") aux extrémités 3'-hydroxyles (3'OH) d'ADN simple- ou double brin. Ainsi, par exemple, l'incubation d'ADN avec la terminale transférase, en présence de désoxycytidine triphosphate (dCTP), conduira à la synthèse, aux 2 extrémités 3'0H de tous les fragments d'ADN, d'une chaîne poly(dC). Dans les conditions adaptées, ce type de marquage présente en particulier l'avantage de marquer uniformément toutes les molécules d'ADN, et donc de ne pas introduire de biais dans la représentativité relative de toutes les molécules d'ADN

Il est pris en compte que l'ADN puisse avoir subi des traitements l'ayant cassé de telle sorte que des extrémités 3'-phosphate (3'P) aient été générées Dans ce cas, l'addition de dNTP aux extrémités de l'ADN par la terminale transférase n'est pas possible. Dans un procédé avantageux selon l'invention, l'ADN est préalablement traité par la nucléase P1 (EC 31301, enzyme extraite en particulier de Penicillium citrinum) qui possède en particulier une activité 3'-nucléotidase enlevant les 3'-phosphates de l'ADN pour générer des extrémités 3'-hydroxyles (3'0H) Ainsi, tous les fragments d'ADN traités seront potentiellement substrats de la terminale transférase.

Les nucléotides incorporés par la terminale transférase peuvent être marqués de façon à être détectés par des techniques classiques telles que l'utilisation d'enzymes susceptibles d'agir sur un substrat chromogène, fluorigène ou luminescent (au moins une des amorces utilisées dans la PCR est par exemple biotinylée et la détection de l'hybridation est réalisée par addition de streptavidine couplée à une enzyme telle que la peroxydase, puis d'un substrat chromogène de l'enzyme), la radioactivité, la fluorescence ou l'électrochimiluminescence Ainsi, de tels fragments, hybridés sur la puce seront identifiés, l'hybridation de l'ADN par la séquence cible apportant la spécificité d'hybridation, l'élongation dNTP marqué permettant la détection au site d'hybridation. Cette réalisation est dépendante de la sensibilité de détection du système puce et de la quantité d'ADN de départ.

Un des aspects remarquable de l'invention est le fait que l'élongation sert de site d'appariement à une amorce de polymérisation, complémentaire de la séquence d'élongation, commune à tous les fragments d'ADN. Par exemple, suite à une élongation poly(dC), une amorce de polymérisation poly(dG) sera utilisée.

Dans un tel procédé avantageux, l'amorce de polymérisation peut être marquée pour la détection de l'hybridation du brin d'ADN nouvellement synthétisé ou bien un nucléotide marqué va être incorporé au cours de la polymérisation pour la détection de l'hybridation du brin d'ADN nouvellement synthétisé. L'incorporation du nucléotide marqué est réalisée en une seule étape de synthèse du brin d'ADN, copie de l'ADN matrice, à partir de l'amorce complémentaire de l'élongation dNTP de la terminale transférase, grâce à l'action d'une quelconque ADN polymérase présentant l'activité enzymatique requise L'incorporation du nucléotide marqué peut également être réalisée au cours d'une succession d'étapes de synthèse du brin d'ADN, copie de l'ADN matrice, à partir de l'amorce complémentaire de l'élongation dNTP de la terminale transférase. Ce marquage peut donc être réalisé directement lors du procédé selon l'invention

L'oligonucléotide complémentaire de l'élongation peut, en plus de la succession d'une même base, comporter à son extrémité 5' une séquence, non complémentaire de la séquence d'élongation, qui lors des étapes de polymérisation de PCR va reconstituer une séquence double brin reconnue par une enzyme de restriction. Bien que cela ne soit pas nécessaire, la présence d'un site de restriction, générant préférentiellement des extrémités cohésives, peut faciliter un clonage des séquences amplifiées.

Le produit généré est ensuite hybridé sur une séquence nucléique de type ADN, ARN ou PNA (Peptide Nucleic Acid), porté sur tout type de support, préférentiellement de type puce à ADN, voire microplaque, et le signal généré par l'éventuelle hybridation mesuré par toute technique possible.

Dans le domaine de l'agro-alimentaire, le procédé de l'invention concerne entre autres les produits de départ comme la viande et les végétaux (ou les produits issus de végétaux sous toutes leurs formes), les produits intermédiaires et les produits finis. Dans d'autres domaines, comme celui de la pharmacie, ceci concerne notamment la lécithine, le sirop de glucose, les produits de fermentation.

### Exemple 1 : Amplification avec une seule amorce de tout l'ADN d'un échantillon issu de plusieurs génomes après une étape de dénaturation, renaturation lente.

Cet exemple est illustré à la figure 1A.

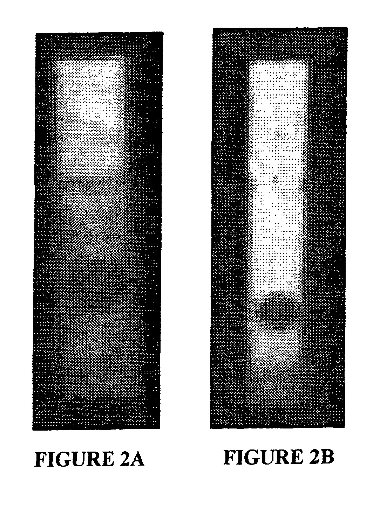

L'ADN de départ est un mélange d'ADN : ADN de soja, ADN de maïs, et ADN humain
**1**^{**ère**} **étape :** Le procédé s'appliquant uniquement à des ADN de petites tailles, les différents ADN sont fragmentés.
   Deux techniques de fragmentation sont utilisées : soit une sonication qui donne des fragments de taille inférieure à 500 pb, soit une digestion par une enzyme de restriction qui coupe l'ADN avec une fréquence relativement élevée et qui produit des extrémités à bords francs (type AluI ou HaeIII) Cette technique donne des fragments plus petits (autour de 250 pb).
**2**^{**ème**} **étape :** La sonication coupe l'ADN de façon aléatoire et produit ainsi des extrémités 3'OH et des extrémités 3'P. La terminale transférase ne permettant d'ajouter des nucléotides qu'aux extrémités 3'OH d'un ADN, les extrémités de l'ADN soniqué sont rendues 3'OH grâce à l'action de la nucléase P1.
   Lorsque l'ADN est fragmenté avec des enzymes de restriction, les extrémités sont 3'OH.
**3**^{**ème**} **étape :** Une séquence homopolymérique est ajoutée à l'extrémité 3' des ADN grâce à la terminale transférase. Pour cela, l'ADN est mis en présence de 120 U de terminale transférase et de 250 µM de dCTP, et incubé 15 minutes à 37°C
   L'ADN est alors purifié sur résine de silice (PCR qiaquick purification kit de Qiagen). L'élongation est vérifiée par hybridation sur bandelette de nitrocellulose l'ADN élongué est fixé aux UV sur la membrane de nitrocellulose puis une amorce homopolymérique (polydG en l'occurrence, le nombre de dG étant de 11, qui sera noté par la suite dG11) biotinylée est mise en contact avec la bandelette et migre par capillarité sur la bandelette. Une révélation colorimétrique de la biotine apparaît au niveau de l'ADN fixé si celui-ci est élongué (figure 2). Dans les conditions d'élongation utilisées, on estime qu'une quinzaine de dCTP ont été incorporés par la terminale transférase.
   L'ADN obtenu est représenté à la figure 1A, III.
**4**^{**ème**} **étape :** Une incubation de 5 minutes à 95°C permet de dénaturer l'ADN obtenu après élongation ; l'amorce homopolymérique dG11 peut alors s'hybrider à la queue homopolymérique précédemment ajoutée et une ADN polymérase permet la synthèse des brins complémentaires. L'ADN obtenu (figure 1A, IV) n'est pas amplifiable tel quel par PCR.
**5**^{**ème**} **étape :** Une dénaturation (5 minutes à 95°C) suivie d'une renaturation lente (passage de 95°C à 35°C avec une rampe de 0,2°C par seconde) permet un réappariement des brins d'ADN et la formation de molécule d'ADN amplifiable par PCR (figure 1A, V).
**6**^{**ème**} **étape** : Après une synthèse des brins complémentaires et une dénaturation, l'ADN peut être amplifié de façon exponentielle par PCR (figure 1A, VI) dans les conditions suivantes :
   tampon d'incubation 1X, 200 µM dNTPs, 1µM oligonucléotide dG11, 3 mM MgCl₂, 2 U Taq DNA polymérase
   cycles d'amplification : 95°C 30 sec / 50°C 2 min / 72°C 1 min 60 cycles
   thermocycleur : Minicycler de MJ Research
   Les étapes 4 à 6 ont également été réalisées sur un thermocycleur à air pulsé (Ligth Cycler de Roche). Pour cela, l'ADN est incubé en présence de 2 µl de DNA Master SYBR GREEN (Roche Boeringher), 0,16 µl d'anticorps anti Taq (Clontech), 5 mM MgCl₂ et 1 µM d'amorce dG11.
   L'ADN est alors soumis au cycles suivants
   Une phase de dénaturation (2 minutes à 95°C)
   Un premier cycle de PCR (95°C 2 sec, 50°C 15 sec, 72°C 28 sec)
   Une nouvelle dénaturation (2 minutes à 95°C) suivie d'une lente renaturation (de 95°C à 35°C avec une rampe de 0,2°C/sec) et 60 cycle de PCR (95°C 2 sec, 50°C 15 sec, 72°C 28 sec).

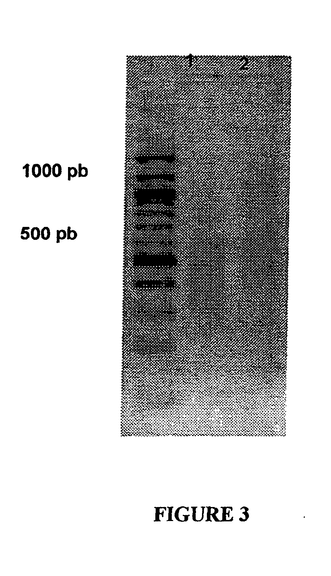

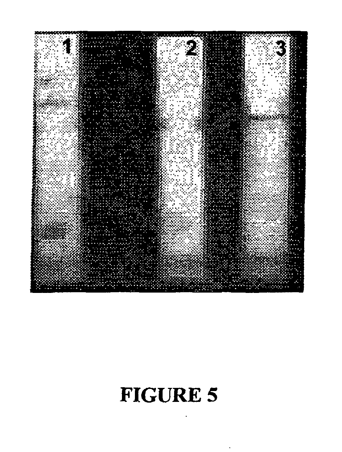

Deux méthodes d'analyse du résultat ont été mises en oeuvre :
Dans un premier temps, l'ADN amplifié est soumis à une électrophorèse sur gel d'agarose 2% (Figure 3).
Dans un deuxième temps, l'amplification est vérifiée par hybridation sur une bandelette de nitrocellulose.
Pour cela, l'amplification est réalisée avec un oligonucléotide dG11 biotinylé Une sonde caractéristique du mélange analysé est déposée sur une bandelette de nitrocellulose et fixée aux UV. Le produit PCR obtenu après amplification est dénaturé et migre par capillarité sur la bandelette de nitrocellulose Si l'amplicon contient la séquence complémentaire à la sonde, une réaction colorimétrique se forme au niveau de la sonde grâce à la révélation de la biotine. Ces résultats sont illustrés à la figure 5.

### Exemple 2 : Amplification avec une seule amorce de tout l'ADN d'un échantillon issu de plusieurs génomes et modifié par deux étapes de terminale transférase.

Cet exemple est illustré à la figure 1B.
Dans cet exemple, l'ADN de départ est le même que dans l'exemple précédent

**1**^{**ère**} **et 2**^{**ème**} **étapes :** Les étapes de fragmentation et de traitement à la nucléase P1 si nécessaire sont identiques à celle de l'exemple 2

**3**^{**ème**} **étape :** Une séquence homopolymérique (polydC en l'occurrence) est ajoutée à l'extrémité 3' de cet ADN et servira d'ancrage à une amorce universelle.
Pour cela, l'ADN est incubé 15 minutes à 37°C en présence de 120 U de terminale transférase et 250 µM de dCTP, puis purifié sur résine de silice (PCR qiaquick purification kit de Qiagen). L'élongation est vérifiée par hybridation sur bandelette de nitrocellulose : l'ADN élongué est fixé aux UV sur la membrane de nitrocellulose puis une amorce homopolymérique (polydG en l'occurrence, le nombre de dG étant de 11) biotinylée est mise en contact avec la bandelette et migre par capillarité sur la bandelette. Une révélation de la biotine apparaît au niveau de l'ADN fixé si celui ci est élongué (figure 2). Dans les conditions d'élongation utilisées, on estime qu'une quinzaine de dCTP ont été incorporée par la terminale transférase

**4**^{**ème**} **étape :** L'ADN élongué est alors dénaturé et une amorce complémentaire de la séquence homoplymérique ajoutée (en l'occurrence une amorce dG11) est hybridée sur l'ADN dénaturé. Une ADN polymérase (Taq DNA polymérase en l'occurrence) est ajoutée pour synthétiser le brin complémentaire. L'ADN obtenu est représenté à la figure 1B, IV.

**5**^{**ème**} **étape :** Une seconde étape de terminale transférase effectuée dans les mêmes conditions que la première est réalisée sur l'ADN élongué et permet ainsi de disposer d'un ADN avec une séquence homopolymérique (en l'occurrence polydC) aux 2 extrémités de l'ADN. Cet ADN est soumis à une dénaturation puis une hybridation avec un oligo dG11 et une polymérisation du brin complémentaire en présence d'une DNA polymérase. L'ADN obtenu est représenté à la figure 1B, V
Une dénaturation suivie de l'hybridation de l'oligonucléotide dG11 et d'une synthèse du brin complémentaire par une ADN polymérase permet d'obtenir un ADN directement amplifiable par PCR avec une amorce homopolymérique (en l'occurrence un dG11) (figure 1B, VI).

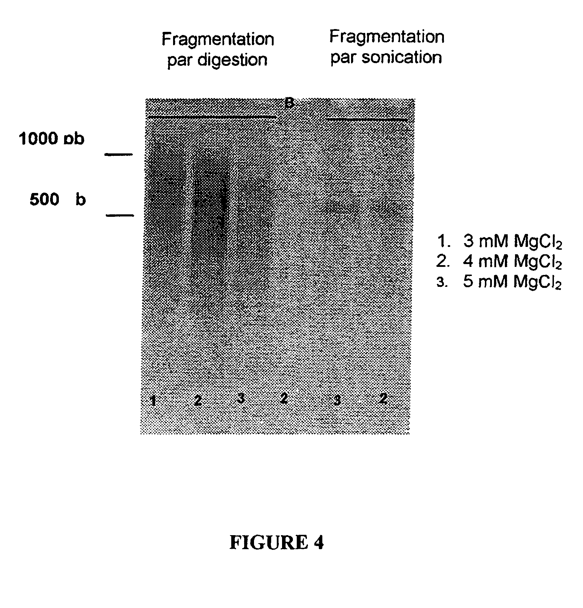

**6**^{**ème**} **étape** : Cette amplification est réalisée sur un thermocycleur minicycler de MJ Research dans les conditions suivantes :
tampon d'incubation 1X, 200 µM dNTPs, 1µM oligonucléotide dG11, 3 mM MgCl₂, 2 U Taq DNA polymérase
cycles d'amplification : 95°C 30 sec / 50°C 2 min / 72°C 1 min 60 cycles
La 6^{ème} étape a également été effectuée sur un thermocycleur à air pulsé (Ligth Cycler de Roche).
Pour cela, l'ADN est incubé en présence de 2 µl de DNA Master SYBR GREEN (Roche Boeringher), 0,16 µl d'anticorps anti Taq (Clontech), 5 mM MgCl₂ et 1 µM d'amorce dG11.
L'ADN est alors soumis à une dénaturation (2 minutes à 95°C), puis à 60 cycles de PCR (95°C 2 sec, 50°C 15 sec, 72°C 28 sec).
Deux méthodes d'analyse du résultat ont été mises en oeuvre :
Dans un premier temps, l'ADN amplifié est soumis à une électrophorèse sur gel d'agarose 2% (Figure 4).
Dans un deuxième temps, l'amplification est vérifiée par hybridation sur une bandelette de nitrocellulose.
Pour cela, l'amplification est réalisée avec un oligonucléotide dG11 biotinylé. Une sonde caractéristique du mélange analysé est déposé sur une bandelette de nitrocellulose et fixée aux UV. Le produit PCR obtenu après amplification est dénaturé et migre par capillarité sur la bandelette de nitrocellulose. Si l'amplicon contient la séquence complémentaire à la sonde, un spot violet se forme au niveau de la sonde grâce à la révélation colorimétrique de la biotine. Ces résultats sont illustrés figure 5.

### Exemple 3 : Pré-amplification pour la détection sur un support solide (une puce à ADN par exemple)

**Etape 1 :** L'ADN, extrait d'un l'échantillon pris sur un produit alimentaire, est réduit à des fragments de taille moyenne d'environ 200 paires de bases en passant la solution dans une aiguille (le nombre de passages de la solution d'ADN à travers l'aiguille et le diamètre de l'aiguille sont deux paramètres qui définissent la taille moyenne de l'ADN en provoquant des cassures aléatoires de l'ADN).
- Les cassures de l'ADN ayant pu générer en particulier des extrémités 3'0H mais également 3'P, l'ADN est ensuite soumis à l'action de la nucléase P1 (EC 31301) qui va enlever les phosphates éventuels aux extrémités 3', générant ainsi des extrémités 3'0H.

**Etape 2 :** L'ADN est ensuite soumis pendant un temps précis à une quantité définie de terminale transférase (EC 27231), en présence d'un désoxyribonucléotide triphosphate (dCTP), ce qui a pour effet d'ajouter aux extrémités une chaîne de désoxyribonucléotide monophosphate.

**Etape 3** : L'ADN est ensuite dénaturé à la chaleur, préférentiellement à une température voisine de 100°C, en présence d'un oligonucléotide de séquence complémentaire à la chaîne polydC ajoutée lors de l'étape 2 et mis à refroidir de façon à permettre l'appariement d'un oligonucléotide poly(dG) (oligod(G)).

**Etape 4 :** L'oligonucléotide va servir d'amorce de synthèse, à partir des 2 extrémités des fragments d'ADN, et la séquence nucléotidique des brins matrices de l'ADN va être synthétisée par une ADN polymérase, en présence des 4 dNTP. A cette étape, on introduit un marquage de l'ADN par l'utilisation de nucléotides marqués.

**Etape 5 :** L'ADN est une nouvelle fois dénaturé, puis renaturé lentement, ce qui a pour effet un appariement des brins complémentaires nouvellement synthétisés portant une extrémité oligo(dG) protubérante en 5'.

**Etape 6 :** Ces extrémités 5' protubérantes sont ensuite complétées par l'action d'une polymérase, en présence de dCTP. L'ensemble du processus peut s'arrêter à cette étape, mais dans cet exemple on poursuit d'avantage l'amplification car l'ADN est à l'état de trace dans l'échantillon

**Etape 7 :** L'ADN ainsi formé sert de matrice à une amplification de type PCR en utilisant comme unique amorce l'oligonucléotide utilisé à l'étape 2 (oligo(dG))

**Etape 8 :** L'ensemble du processus ayant eu pour effet d'amplifier d'un facteur au minimum supérieur à 2, la totalité de l'ADN de l'échantillon, en ayant également incorporé un marqueur dans cet ADN, celui ci est ensuite dénaturé et des séquences spécifiques sont recherchées par hybridation sur une sonde de capture fixée sur une puce à ADN (telle que les puces commercialisées par Affymetrix, Cis Bio International / LETI). On détecte ainsi la présence ou l'absence d'une contamination par une bactérie (par exemple, la bactérie du botulisme en utilisant des sondes de capture spécifiques à cette bactérie).

### Exemple 4 : Pré-amplification aspécifique et amplification spécifique.

L'ensemble des produits d'amplification aspécifiques subissent une nouvelle amplification spécifique à l'aide d'amorces spécifiques. Dans ce cas, le marquage peut avoir lieu à cette étape et les produits d'amplification spécifiques être détectés de la même façon que décrit ci-dessus. Plus généralement, la nouvelle amplification peut également être réalisée à l'aide d'amorces non spécifiques, de séquences dégénérées, ou de type microsatellites. Le procédé de l'invention est dans cet exemple une méhode de pré-amplification de la totalité de l'ADN de l'échantillon avant une amplification plus spécifique de séquences d'intérêts. On peut ainsi détecter des fragments de séquences à l'état de trace, même à partir d'échantillons difficiles à analyser.

### Exemple 5 : Analyse quantitative.

Dans des conditions de PCR quantitative (en particulier quand le nombre de cycles d'amplification est tel que la quantité de chaque produit d'amplification est proportionnelle à la quantité d'ADN de départ), en utilisant l'amorce d'amplification marquée, le signal lu après hybridation soit sur un appareil à PCR quantitative tel que le LightCycler™ (Roche Diagnostics), l'Abi Prism 7700™ (Perkin Elmer) ou l'*i* cycler™ (Bio-Rad), soit sur un support permettant une PCR quantitative tel que une puce à ADN par exemple, permettra de quantifier, en absolu ou en relatif selon les conditions, la quantité d'une séquence précise d'ADN dans l'échantillon de départ L'hybridation sur un oligonucléotide de capture complémentaire de l'oligonucléotide utilisé dans le procédé (ou du nucléotide utilisé dans l'élongation), selon l'orientation de ces oligonucléotides de capture, permet une détection de l'ensemble des produits amplifiés et autorise donc la quantification relative de l'hybridation spécifique d'une séquence particulière.

## Revendications

1. Procédé d'amplification de la totalité des fragments d'ADN double brin d'un échantillon, comprenant les étapes suivantes :
a) Extraction de l'ADN et réduction le cas échéant de la taille des fragments d'ADN extraits, par cassure physique ou enzymatique de manière à obtenir une longueur moyenne comprise entre 25 et 500 pb.
b) Addition d'un oligonucléotide poly(dX) aux extrémités 3' des fragments d'ADN par une terminale transférase.
c) Dénaturation et appartement avec une amorce poly(dY) complémentaire du poly(dX) de l'étape b).
d) Synthèse par une ADN polymérase en présence des quatre dNTP.
e) Obtention d'un ADN amplifiable consistant soit en une dénaturation, puis une lente renaturation pour apparier les brins complémentaires synthétisés à l'étape d) portant des extrémités protubérantes poly(dY), soit en une seconde étape de terminale transférase effectuée dans les mêmes conditions que rétape b).

2. Procédé selon la revendication 1 **caractérisé en ce que** l'étape e) consiste en une dénaturation, puis une lente renaturation pour apparier les brins complémentaires synthétisés à l'étape d) portant des extrémités protubérantes poly(dY).

3. Procédé selon la revendication 1 **caractérisé en ce que** l'étape e) consiste en une seconde étape de terminale transférase effectuée dans les mêmes conditions que l'étape b).

4. Procédé selon l'une des revendications 1 à 3 **caractérisé en ce qu'**on amplifie l'ADN obtenu à l'étape e), ladite amplification comprenant les étapes suivantes :
f) Synthèse par une ADN polymérase des poly(dX) complémentaires desdites protubérances, éventuellement en présence uniquement de dXTP.
g) Succession de cycles de type PCR consistant en la dénaturation, l'appariement de l'amorce poly(dY), et la synthèse.

5. Procédé selon l'une des revendications 1 à 4 **caractérisé en ce que** les fragments d'ADN possèdent une longueur moyenne comprise entre 100 et 300 pb, de préférence égale à 200 pb

6. Procédé selon l'une des revendications 1 et 2 **caractérisé en ce que** la renaturation lente est effectuée par un passage d'une température comprise entre 85°C et 105°C à une température comprise entre 45° et 25°C, de préférence de 95°C à 35°C avec une rampe de température allant environ de 0,5°C à 0,05°C par seconde, de préférence de 0,2°C par seconde.

7. Procédé selon l'une des revendications 1 à 6 **caractérisé en ce que** des extrémités 3'-OH libres sont générées par la nucléase P1 avant l'addition d'un oligonucléotide poly(dX) aux extrémités 3' desdits fragments.

8. Procédé selon l'une des revendications 1 à 7 **caractérisé en ce que** l'oligonucléotide poly(dX) ou poly(dY) est marqué radioactivement, par un groupe fluorescent ou chimiluminescent ou en utilisant un système permettant une révélation par colorimétrie, notamment le système biotine-streptavidine-couplée à une enzyme réagissant avec un substrat chromogène, fluorigène ou luminescent.

9. Procédé selon l'une des revendications 1 à 8 **caractérisé en ce qu'**au moins un nucléotide marqué est incorporé au cours d'une des étapes de synthèse.

10. Procédé de caractérisation d'un échantillon consistant en l'hybridation des fragments d'ADN obtenus à partir d'un procédé selon l'une des revendications 8 et 9 sur une ou plusieurs séquences nucléique de type ADN, ARN, ou PNA portée(s) sur un support solide, et en la visualisation du signal émis par le ou les fragment(s) hybridé(s)

11. Procédé selon la revendication 10 **caractérisé en ce que** le support solide est une puce à ADN, ARN ou PNA, une microplaque, ou un film de nitrocellulose.

12. Kit de détection permettant de mettre en oeuvre le procédé selon l'une des revendications 1 à 11, **caractérisé en ce qu'**il comprend des oligonucléotides homopolymériques, une terminale transférase, la nucléase P1, des sondes spécifiques permettant la détection des molécules recherchées ou des puces à ADN, ARN ou PNA permettant ladite détection.

13. Utilisation du procédé selon l'une des revendication 1 à 11 pour l'identification d'un produit, d'une substance, et/ou d'une matière, à l'état naturel ou qui a subi des traitements, des transformations, et/ou des conditionnements, pour l'identification de son origine et/ou de son appartenance.

14. Utilisation selon la revendication 13 pour la détection de la présence d'organismes génétiquement modifiés (OGM) ou de traces d'OGM dans un échantillon.

15. Utilisation du procédé selon l'une des revendication 1 à 11 pour l'identification et/ou la quantification de contaminants dans un produit.

16. Utilisation selon la revendication 15 **caractérisée en ce que** le produit est une sécrétion humaine, éventuellement à l'état de trace.

17. Utilisation selon la revendication 15 **caractérisée en ce que** le produit est un extrait végétal, ou animal, éventuellement transgénique.

18. Utilisation selon la revendication 15 **caractérisée en ce que** le produit est un produit agro-alimentaire ou pharmaceutique.

19. Utilisation selon l'une des revendications 15 à 18 **caractérisée en ce que** le contaminant est un micro-organismes telle qu'une bactérie, un virus, ou un champignon.

## Claims

1. Method for amplifying all of the doublestranded DNA fragments of a sample, comprising the following steps:
a) extraction of the DNA and reduction, where appropriate, of the size of the DNA fragments extracted, by physical or enzymatic cleavage so as to obtain a mean length of between 25 and 500 bp.
b) addition of a poly(dX) oligonucleotide to the 3' ends of the DNA fragments using a terminal transferase.
c) denaturation and annealing with a poly(dY) primer complementary to the poly(dX) of step b).
d) synthesis using a DNA polymerase in the presence of the four dNTPs.
e) production of a DNA which can be amplified, consisting either of denaturation and then of slow renaturation in order to anneal the complementary strands synthesized in step d) carrying protruding poly(dY) ends, or of a second terminal transferase step carried out under the same conditions as step b).

2. Method according to Claim 1, **characterized in that** step e) consists of denaturation and then of slow renaturation in order to anneal the complementary strands synthesized in step d) carrying protruding poly(dY) ends.

3. Method according to Claim 1, **characterized in that** step e) consists of a second terminal transferase step carried out under the same conditions as step b).

4. Method according to one of Claims 1 to 3, **characterized in that** the DNA obtained in step e) is amplified, said amplification comprising the following steps:
f) synthesis, using a DNA polymerase, of the poly(dx)s complementary to said protrusions, optionally in the presence only of dXTP.
g) series of PCR-type cycles consisting of denaturation, annealing of the poly(dY) primer and synthesis.

5. Method according to one of Claims 1 to 4, **characterized in that** the DNA fragments have a mean length of between 100 and 300 bp, preferably equal to 200 bp.

6. Method according to either of Claims 1 and 2, **characterized in that** the slow renaturation is carried out by dropping from a temperature of between 85°C and 105°C to a temperature of between 45° and 25°C, preferably from 95°C to 35°C, with a temperature ramp ranging approximately from 0.5°C to 0.05°C per second, preferably of 0.2°C per second.

7. Method according to one of Claims 1 to 6, **characterized in that** free 3'-OH ends are generated using the P1 nuclease before adding a poly(dX) oligonucleotide to the 3' ends of said fragments.

8. Method according to one of Claims 1 to 7, **characterized in that** the poly(dX) or poly(dY) oligonucleotide is labelled radioactively, with a fluorescent or chemiluminescent group, or using a system allowing revelation by colorimetry, in particular the system biotin-streptavidin coupled to an enzyme which reacts with a chromogenic, fluorigenic or luminescent substrate.

9. Method according to one of Claims 1 to 8, **characterized in that** at least one labelled nucleotide is incorporated during one of the synthesis steps.

10. Method for **characterizing** a sample, consisting in hybridizing the DNA fragments obtained using a method according to either of Claims 8 and 9 to one or more nucleic sequences of DNA, RNA or PNA type carried on a solid support, and in visualizing the signal emitted by the hybridized fragment(s).

11. Method according to Claim 10, **characterized in that** the solid support is a DNA, RNA or PNA chip, a microplate or a nitrocellulose film.

12. Detection kit making it possible to implement the method according to one of Claims 1 to 11, **characterized in that** it comprises homopolymeric oligonucleotides, a terminal transferase, the P1 nuclease, specific probes allowing the detection of the molecules sought, or DNA, RNA or PNA chips allowing said detection.

13. Use of the method according to one of Claims 1 to 11, for identifying a product, a substance and/or a material, in unmodified form or which has undergone treatments, transformations and/or conditioning, or for identifying its origin and/or its family.

14. Use according to Claim 13, for detecting the presence of genetically modified organisms (GMOs) or of traces of GMO in a sample.

15. Use of the method according to one of Claims 1 to 11, for identifying and/or quantifying contaminants in a product.

16. Use according to Claim 15, **characterized in that** the product is a human secretion, possibly in trace amounts.

17. Use according to Claim 15, **characterized in that** the product is an extract of a plant, or an animal, possibly transgenic.

18. Use according to Claim 15, **characterized in that** the product is an agro-foods or pharmaceutical product.

19. Use according to one of Claims 15 to 18, **characterized in that** the contaminant is a microorganism such as a bacterium, a virus or a fungus.

## Patentansprüche

1. Verfahren zur Amplifizierung der Gesamtheit der doppelsträngigen DNA-Fragmente einer Probe, welches die folgenden Schritte umfasst:
a) Extraktion der DNA und gegebenenfalls erfolgende Verringerung der Größe der extrahierten DNA-Fragmente durch physikalisches oder enzymatisches Zerkleinern oder Schneiden dergestalt, dass eine mittlere Länge zwischen 25 und 500 bp erhalten wird,
b) Anfügen eines poly(dX)-Oligonukleotids an die 3'-Enden der DNA-Fragmente durch eine terminale Transferase,
c) Denaturierung und Paarbildung mit einem zu der poly(dX)-Struktur des Schritts b) komplementären poly(dY)-Primer,
d) Synthese durch eine DNA-Polymerase in Gegenwart der vier dNTPs,
e) Gewinnung einer amplifizierbaren DNA, bestehend entweder in einer Denaturierung, dann einer langsamen Renaturierung, um eine Paarbildung der in Schritt d) synthetisierten komplementären Stränge, die überhängende poly(dY)-Enden tragen, zu bewirken, oder in einem zweiten terminale Transferase-Schritt, der unter den gleichen Bedingungen wie Schritt b) ausgeführt wird.

2. Verfahren nach Anspruch 1, **dadurch gekennzeichnet, dass** der Schritt e) in einer Denaturierung, dann einer langsamen Renaturierung, um eine Paarbildung der in Schritt d) synthetisierten komplementären Stränge, die überhängende poly(dY)-Enden tragen, zu bewirken, besteht.

3. Verfahren nach Anspruch 1, **dadurch gekennzeichnet, dass** der Schritt e) in einem zweiten terminale Transferase-Schritt besteht, der unter den gleichen Bedingungen wie Schritt b) ausgeführt wird.

4. Verfahren nach einem der Ansprüche 1 bis 3, **dadurch gekennzeichnet, dass** man die in Schritt e) erhaltene DNA amplifiziert, wobei diese Amplifizierung die folgenden Schritte umfasst:
f) Synthese der zu den überhängenden Sequenzen komplementären poly(dX)-Sequenzen durch eine DNA-Polymerase, gegebenenfalls in alleiniger Gegenwart von dXTP,
g) Aufeinanderfolge von Cyclen vom PCR-Typ, bestehend aus Denaturierung, Anhybridisierung des poly(dY)-Primers und Synthese.

5. Verfahren nach einem der Ansprüche 1 bis 4, **dadurch gekennzeichnet, dass** die DNA-Fragmente eine mittlere Länge zwischen 100 und 300 bp, vorzugsweise von 200 bp aufweisen.

6. Verfahren nach einem der Ansprüche 1 und 2, **dadurch gekennzeichnet, dass** die langsame Renaturierung durch einen Übergang von einer Temperatur zwischen 85°C und 105°C zu einer Temperatur zwischen 45° und 25°C, vorzugsweise von 95°C zu 35°C, mit einer Temperaturänderung, die von etwa 0,5°C bis 0,05°C pro Sekunde geht, vorzugsweise von 0,2°C pro Sekunde, bewirkt wird.

7. Verfahren nach einem der Ansprüche 1 bis 6, **dadurch gekennzeichnet, dass** vor dem Anfügen eines poly(dX)-Oligonukleotids an den 3'-Enden des Fragments durch die P1-Nuklease freie 3'-OH-Enden erzeugt werden.

8. Verfahren nach einem der Ansprüche 1 bis 7, **dadurch gekennzeichnet, dass** das poly(dX)- oder poly(dY)-Oligonukleotid radioaktiv, durch eine fluoreszierende oder chemolumineszierende Gruppe oder durch Einsatz eines Systems, das einen Nachweis durch Kolorimetrie erlaubt, insbesondere des Systems Biotin-Streptavidin, gekoppelt mit einem Enzym, das mit einem chromogenen, fluorigenen oder lumineszierenden Substrat reagiert, markiert ist.

9. Verfahren nach einem der Ansprüche 1 bis 8, **dadurch gekennzeichnet, dass** wenigstens ein markiertes Nukleotid während eines der Syntheseschritte eingebaut wird.

10. Verfahren zur Charakterisierung einer Probe, bestehend aus der Hybridisierung der ausgehend von einem Verfahren gemäß einem der Ansprüche 8 oder 9 erhaltenen DNA-Fragmente an eine oder mehrere Nukleinsäuresequenzen vom DNA-, RNA- oder PNA-Typ, die sich auf einem festen Träger befindet bzw. befinden, und aus der Sichtbarmachung des Signals, das von dem oder den hybridisierten Fragment(en) ausgesandt wird.

11. Verfahren nach Anspruch 10, **dadurch gekennzeichnet, dass** der feste Träger ein DNA-, RNA- oder PNA-Chip, eine Mikroplatte oder ein Nitrocellulosefilm ist.

12. Nachweiskit, der es ermöglicht, das Verfahren nach einem der Ansprüche 1 bis 11 auszuführen, **dadurch gekennzeichnet, dass** er homopolymere Oligonukleotide, eine terminale Transferase, die Nuklease P1, spezifische Sonden, die den Nachweis der gesuchten Moleküle erlauben, oder DNA-, RNA- oder PNA-Chips, die diesen Nachweis erlauben, umfasst.

13. Verwendung des Verfahrens nach einem der Ansprüche 1 bis 11 zur Identifizierung eines Produkts, einer Substanz und/oder eines Materials in natürlichem Zustand, oder das bzw. die Behandlungen, Umwandlungen und/oder Konditionierungen unterworfen worden ist, zur Identifizierung von dessen bzw. deren Herkunft und/oder dessen bzw. deren Zugehörigkeit.

14. Verwendung nach Anspruch 13 für den Nachweis der Anwesenheit von genetisch modifizierten Organismen (GMO) oder von Spuren von GMO in einer Probe.

15. Verwendung des Verfahrens nach einem der Ansprüche 1 bis 11 für die Identifizierung und/oder die Quantifizierung von Verunreinigungen in einem Produkt.

16. Verwendung nach Anspruch 15, **dadurch gekennzeichnet, dass** das Produkt eine menschliche Absonderung, gegebenenfalls im Zustand einer Spur, ist.

17. Verwendung nach Anspruch 15, **dadurch gekennzeichnet, dass** das Produkt ein gegebenenfalls transgener pflanzlicher oder tierischer Extrakt ist.

18. Verwendung nach Anspruch 15, **dadurch gekennzeichnet, dass** das Produkt ein agrarwirtschaftliches oder pharmazeutisches Produkt ist.

19. Verwendung nach einem der Ansprüche 15 bis 18, **dadurch gekennzeichnet, dass** die Verunreinigung ein Mikroorganismus, wie ein Bakterium, ein Virus oder ein Pilz, ist.
